# EUROPÄISCHE PATENTANMELDUNG

(11) **EP 2 560 004 A1**
(43) Veröffentlichungstag der Anmeldung: **20.02.2013**
(21) Anmeldenummer: 11178193.6
(22) Anmeldetag: 19.08.2011
(51) Int. Cl.: G01N 33/543

(54) **Neues PoC-Testsystem und Verfahren**

(71) Anmelder: DST Diagnostische Systeme & Technologien GmbH, 19061 Schwerin (DE)
(72) Erfinder: Rübenhagen, René, 19061 Schwerin (DE); Dangers, Marc, 19053 Schwerin (DE)
(74) Vertreter: Simandi, Claus

(57) **Zusammenfassung**

Die vorliegende Erfindung betrifft ein Testsystem oder Assaysystem (Nachweissystem)und Testverfahren vorzugsweise im Gebrauch für den Point-of-Care (PoC) Bereich.

## Beschreibung

Die vorliegende Erfindung betrifft ein Testsystem oder Assaysystem (Nachweissystem)und Testverfahren vorzugsweise im Gebrauch für den Point-of-Care (PoC) Bereich.

In der Forschung und in-vitro Diagnostik im humanen, veterinären, Lebensmittel- oder in einer Vielzahl weiterer Anwendungsbereiche dienen analytische Tests zur qualitativen oder/und quantitativen Bestimmung von Molekülen, Analyten bzw. deren Aktivität oder Zusammensetzung. Die Testresultate liefern unterschiedliche Aussagen wie beispielsweise diagnostische Parameter zur Identifikation von Krankheiten, Herkunft von Lebensmitteln oder Wirksamkeit eines Medikaments.

Heute dominieren die bekannten Methoden der DNA- und Proteinanalytik in den verfügbaren Tests. Insbesondere Immunoassays stellen Verfahren dar, bei denen Antikörper zur spezifischen Bindung von ausgesuchten, ausreichend großen Zielmolekülen fast beliebiger Art, beispielsweise Biomarkern, eingesetzt werden.

Schnelltests liefern Messwerte innerhalb von wenigen Minuten oder Stunden nach Beginn des Tests, und nahe am Ort der Probennahme und ohne Notwendigkeit, die Probe einzusenden (Point-of-Care (PoC)).

Bekannte standardisierte Testverfahren sind Lateral-Flow-Test (LFT), Flow-Through-Test (FTT), Agglutinations-Test (AT) oder Solid-Phase-Test (SPT). Alle diese Verfahren weisen Analyte direkt visuell nach. Für solche Tests gibt es zusätzlich kompakte Auslesegeräte, die auch quantitative Ergebnisse liefern.

Bekannte Assayverfahren der in-vitro Diagnostik sind Immunoassays (IA), insbesondere Enzyme-linked Immunoassays (EIA), oder "binding assay" ("sandwich") (Siehe z.B. EP0171150B1, EP 0063810B1). Darüber hinaus sei auf das Schrifttum von Roger P. Ekins (z.B. WO 8401031 u.v.a.) verwiesen.

Membrangestütze Bindungsassays, insbesondere von IgE aus Blut an Allergenen sind seit Ende der 1980-iger Jahre bekannt, so etwa aus CHEMICAL ABSTRACTS, vol. 101, no. 25, 17th December 1984, page 578, abstract no. 228190b, Columbus, Ohio, US; B.J. WALSH et al.: "Allergen discs prepared from nitrocellulose: detection of IgE binding to soluble and insoluble allergens", & J. IMMUNOL. METHODS 1984, 73(1), 139-45. Einige Testsysteme, die auf dem Prinzip des Lateral Flow beruhen, sind als Allergieschnelltest kommerziell erhältlich.

Für den PoC - Bereich ist beispielsweise der käuflich erhältliche Fast-Check-PoC der Anmelderin beschrieben (siehe EP1718970), der auf Basis eines membrangestützten Bindungsassays zum Allergienachweis aus Vollblut eingesetzt werden kann.

Ferner ist ein Schnelltest der Anmelderin in W02011/000959 beschrieben, bei dem eine Membran gezielt zwischen den Nachweisstellen benetzt wird, um die Testreagenzien besonders effektiv und parallel in spezifische Regionen der zuvor mit dem zu analysierenden Vollblut beladene Membran einzubringen.

Es besteht jedoch ein großer Bedarf an verbesserten Schnelltests.

Insbesondere die gleichmäßige Beladung der einzelnen Nachweisplätze mit Probenflüssigkeit (Analyt) ist nach wie vor verbesserungsfähig, als auch eine effiziente Spülung aller Nachweisplätze. Beides ist jedoch Voraussetzung für reproduzierbare und qualitativ hochwertige Ergebnisse.

Daher ist es Aufgabe der vorliegenden Erfindung für ein PoC-Testsystem ein verbessertes Verfahren zum Beladen eines Trägers mit Probenflüssigkeit zum Nachweis von Analyten mittels Rezeptormolekülen bereitzustellen.

Überraschender Weise wird diese Aufgabe durch den Anspruch 1 gelöst. Erfindungsgemäß ist ein Testverfahren vorgesehen, zum Nachweis mindestens einem Analyten aus einer Probenflüssigkeit,
wobei in einer Probenkammer
a. mindestens ein Rezeptormolekül auf einer ersten Oberfläche eines Trägers fixiert ist, und rückseitig eine zweite Oberfläche ausgebildet ist,
b. der Träger für eine Probenflüssigkeit teilweise durchlässig ist,
c. über der ersten und zweiten Oberfläche jeweils zumindest ein Freivolumen ausgebildet ist, welches von einer Kammerwand begrenzt ist,
d. die Probenkammer mindestens zwei Öffnungen aufweist, wobei durch eine Öffnung eine Probenflüssigkeit entlang einem Strömungsgradienten über die erste und zweite Oberfläche zu der zweiten Öffnung fließt,
e. das Freivolumen aus c.) zumindest teilweise mit einer Flüssigkeitssäule ausgebildet ist,
f. wobei der Strömungsgradient entlang der ersten und zweiten Oberfläche mittels Druckbeaufschlagung erfolgt und die Flüssigkeitssäule aus d.) mitführt.
Nachstehend das "erfindungsgemäße Verfahren".

Das erfindungsgemäße Verfahren erlaubt besonders vorteilhaft das Beladen eines Trägers mit Probenflüssigkeit zum Nachweis von Analyten. Hierbei wird eine qualitative Verbesserung durch die zeitweise Ausbildung einer Flüssigkeitssäule entlang dem Strömungsgradienten über den Träger und der relevanten Nachweisplätze mit den Rezeptormolekülen erhalten.

Erfindungsgemäß wesentlich ist, dass ein hinreichender Strömungsgradient bezüglich beider Trägeroberflächen, d.h. an der ersten und zweiten Oberfläche entsteht, wobei vorteilhaft ein Transport eines wesentlichen Anteils, zumindest jedoch die Hälfte der Probenflüssigkeit außerhalb des Trägers erreicht werden kann. Dies gilt vor allem für jenen Bereich des Trägers, der beim Einströmen der Probenflüssigkeit zuerst erreicht wird und auf dem sich auf einem Nachweisplatz mindestens ein Rezeptormolekül befindet. Bevorzugt werden auch andere Lösungen, wie Spüllösung, im Wesentlichen außerhalb des Trägers transportiert.

Erfindungsgemäß bildet sich die Flüssigkeitssäule von der ersten oder zweiten Oberfläche zur gegenüberliegenden Kammerwand (Kammerdeckel oder Kammerboden) aus.

In einer weiteren bevorzugten Ausführungsform beträgt der Abstand der ersten oder zweiten Oberfläche des Trägers zur Kammerwand 10 µm und mehr, wobei über der ersten und / oder zweiten Oberfläche eine Flüssigkeitssäule im Freivolumen steht, sobald der Strömungsgradient entsteht.

Bevorzugt ist jedoch ein Abstand von 10 bis 1.500 µm (1,5 mm) zwischen der ersten und zweiten Oberfläche jeweils zur Kammerwand, wobei vorteilhaft kein Abreißen des Strömungsgradienten erfolgen kann, weiterhin bevorzugt ist ein Abstand von 80 µm bis 350 µm, und besonders bevorzugt ist ein Abstand von 120 µm bis 200 µm.

Weiterhin kann es bevorzugt sein, dass beide Abstände der jeweils ersten Oberfläche oder zweiten Oberfläche des Trägers unterschiedlich sind und insbesondere beispielsweise 170 µm zum Kammerdeckel (Kammerwand, oben) und beispielsweise 150 µm zum Kammerboden (Kammerwand, unten) hin betragen. Die Variation ist abhängig vom gewünschten Strömungsgradienten in Abhängigkeit der Probenflüssigkeit.

Bevorzugt ist ferner, dass der Träger länglich zugeschnitten ist und an den beiden Längsseiten fest an der Probenkammer befestigt ist. Bevorzugt ist es, die Längsseiten zwischen zwei Rändern der Halbschalen, die eine Probenkammer und den Freiraum bzw. Freivolumen über und unter der Membran bilden, zu klemmen. Der Träger kann ebenfalls an den Längsseiten geklebt sein. Eine Kombination aus Klemmung und Klebung ist ebenfalls möglich.

In einer weiteren bevorzugten Ausführungsform der Erfindung enthält die Testvorrichtung eine weitere Kammer neben der Probenkammer, die mit der Auslassöffnung der Probenkammer fluidisch verbunden ist, und in der die zugegebenen Flüssigkeiten (Probenflüssigkeit, Spülflüssigkeit, Lösungen, etc.) gesammelt werden, nachdem sie durch die Auslassöffnung der Probenkammer geströmt sind. Diese zweite (Neben-)Kammer kann mit saugfähigem Material, beispielsweise Watte, Zellstoff, Polyacrylate oder funktionalisierte Polyacrylate in bekannter Weise ausgestattet sein. Bevorzugt ist ein Kammervolumen von mindestens 1,0 ml, insbesondere 5 - 10 ml. In einer weiteren bevorzugten Ausführungsform der Erfindung wird die Probenflüssigkeit nicht direkt auf den Träger appliziert, sondern über einen (Proben)Kanal in der Testvorrichtung in die Probenkammer mit Druckbeschlag eingegeben oder mittels Unterdruck eingesaugt. In einer bevorzugten Ausführungsform ist der (Proben)kanal parallel zum Träger ausgerichtet bzw. in Richtung des zu bildenden Strömungsgradienten.

Bevorzugt ist weiterhin die Verwendung von Probenvolumina oder Lösungsvolumina, die größer sind als das Flüssigkeitsvolumen, dass der Träger aufnehmen kann, oder größer sind als das Gesamtvolumen des Trägers in feuchtem Zustand (siehe Beispiele).

Der Strömungsgradient kann mittels einer Druckbeaufschlagung erzeugt werden, wobei die Probenflüssigkeit in die Probenkammer druckbeaufschlagt wird. Eine geeignete Druckbeaufschlagung kann manuell, insbesondere durch eine Spritze, Ampulle oder maschinell, insbesondere durch eine Pumpe oder einen Balg erzeugt werden.

Ein beispielhafter geeigneter Strömungsgradient kann wie folgt erreicht werden:
Eine ausreichende Druckbeaufschlagung ist zum Beispiel 150 hPa über der Kammer, bei einem typischen Probenflüssigkeitsvolumen von 95 µl, falls die Höhe des Freivolumens 320 µm, seine Breite 3,5 mm, seine Länge 50,5 mm und die angestrebte Befüllzeit 5s betragen. Bei einer zehntel Befüllzeit also 0,5s beträgt der notwendige Druck etwa 10-fach also 1.500 hPa unter sonst gleichen Bedingungen. Wird Unterdruck an der Auslassöffnung angelegt, beträgt die Befüllzeit mindestens 0,75s bei ansonsten gleichen Bedingungen.

Eine weitere bevorzugte mittlere Strömungsgeschwindigkeit der Probenflüssigkeit in der Kammer beträgt 0,1 m/s, die beispielsweise mit einer 1.000 µl Einmalspritze bei 4 Kanälen manuell bequem erreicht werden kann und einer Befülldauer von 5s entspricht (siehe Beispiel).

Weiterhin ist bevorzugt, dass zum Beispiel bei einer Probenflüssigkeit mit einem Volumen von 95 µl das Freivolumen über dem Träger eine Länge des Trägers von 50 mm und eine Breite von 3,5 mm aufweist, bei einer Gesamthöhe von 320 µm (Kammerboden zu Kammerdecke), wobei der Abstand der ersten Oberfläche zur Kammerdecke 170 µm beträgt und der Abstand der zweiten Oberfläche zum Kammerboden 150 µm beträgt.

Der maximale Über- oder Unterdruck und Fließwiderstand der Probenkammer sind auch bei anderen Dimensionen der Probenkammer und des Trägers in der Weise zu bemessen, dass eine Probenflüssigkeit die Auslassöffnung gemäß dem erfindungsgemäßen Verfahren in weniger als 5 Sekunden, vorzugsweise in weniger als 2 Sekunden erreicht.

Weiterhin ist vorteilhaft, dass der Durchmesser der Einlassöffnung bei einem gesetzten Probenflüssigkeitsvolumen von z.B. 95 µl 0,3 mm und der der Auslassöffnung 1,2 mm beträgt, wobei die Wegstrecke auf dem Träger bei gegebener Strömungsgeschwindigkeit vorzugsweise 50,5 mm beträgt. Andere Dimensionen sind entsprechend zu extrapolieren anhand der weiteren bevorzugten Ausführungsformen.

Daher betrifft die Erfindung in einer bevorzugten Ausführungsform eine optimierte Probenkammer bei einer Eingabe einer Probenflüssigkeit von 80 - 120 µl, wobei die Länge des Trägers 40 - 60 mm, die Breite des Trägers 2,0 - 5 mm, und der Einlassdurchmesser 0,15 mm bis 0,45, der Auslassdurchmesser 1 mm bis 1,5 mm, die Gesamthöhe der Probenkammer 260 µm bis 450 µm betragen.

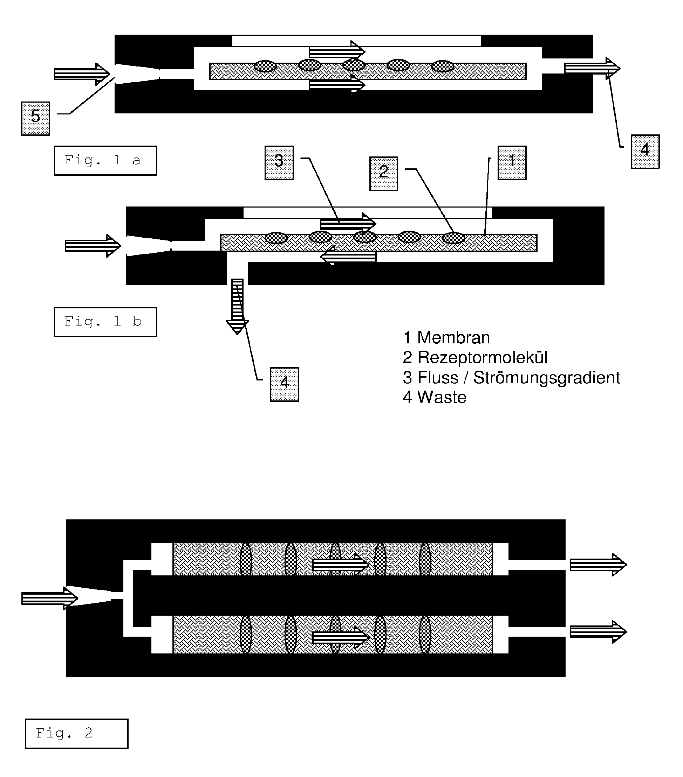

In weiteren Ausführungsformen kann der Strömungsgradient wahlweise a.) parallel an der ersten und zweiten Oberfläche des Trägers geführt werden oder b.) antiparallel an der ersten und zweiten Oberfläche des Trägers geführt werden (siehe Abb. 1a und 1b).

Besonders vorteilhaft ist eine Ausführungsform, bei der die Strömung zunächst entlang der Oberfläche des Trägers geführt wird, um eine Kante des Trägers herum oder durch eine Führung auf die zweite Oberfläche des Trägers und folglich in Gegenrichtung entlang zur zweiten Öffnung der Probenkammer (Abb. 1b). Bei dieser Ausführungsform wird die Strömung nicht geteilt bzw. an der Trägerfußkante gebrochen und wird entlang beider Oberflächen des Trägers geleitet. Es besteht ein geringes Risiko einer Strömungsinhomogenität an der Bifurkation. Zudem ist bei dieser Ausführungsform bei gleichem Volumen der Probenkammer die Strömungsgeschwindigkeit der Lösung doppelt so hoch, wie bei der Ausführungsform mit gleichgerichteter Strömung. Bei gleichem Volumen an eingesetzter Flüssigkeit wird zudem aufgrund der höheren Strömungsgeschwindigkeit eine bessere Spülwirkung erreicht.

In einer weiteren bevorzugten Ausführungsform können mehrere Probenkammern (z.B. 1 bis 10) parallel geschaltet sein und aus einem Probenkanal gespeist werden (s. Abb. 2).

Daher betrifft die Erfindung ein Verfahren, wobei mehrere Probenkammern parallel aus einem Probenkanal mit mindestens einer Probenflüssigkeit versorgt werden.

Im Rahmen dieser Erfindung bedeutet der Begriff "Rezeptormolekül" Substanzen, wie Peptide, Proteine, Nukleinsäuren, Enzyme, Liganden, Rezeptoren, Antikörper oder Antigene, DNA, RNA, PNA, ebenfalls Moleküle nicht biochemischen Ursprungs, wie beispielsweise synthetische Moleküle. Die Testreagenzien können Kombinationen dieser Molekülsorten enthalten oder deren Fragmente, Konjugate, modifizierte, etwa glykolisierte oder phosphorylierte Formen. Lipide und Zucker sind ebenfalls erfindungsgemäß umfasst. Ferner können Naturstoffe und natürliche Extrakte in Testreagenzien vorkommen, wie dies bei Allergietests oder Nahrungsmittelunverträglichkeittests häufig der Fall ist. Mindestens ein Rezeptormolekül bindet mit mindestens einem Analyten aus der Probenflüssigkeit. Daher betrifft die Erfindung ebenfalls einen Bindungsassay, wobei mittels dem erfindungsgemäßen Verfahren besonders reproduzierbare Ergebnisse erhalten werden können.

Im Sinne dieser Erfindung besteht der Träger aus einem festen Material, aufweisend eine erste und zweite Oberfläche, ganz oder teilweise aus einer gelartigen, porösen, siebartigen, permeablen oder semipermeablen Membran, Dialysemembran, insbesondere einer beschichteten oder unbeschichteten Membran. Beispielsweise solche wie Nitrozellulose, PVDF, Zeolith, Sintermaterialien, insbesondere Polyethylenoxid, nachträglich mit Mikrokanälen versehenen Materialien wie Silizium, Glas oder Kunststoffen, die durch Laserbohren, Ionenbeschuß oder Ätzen durchlässig gemacht wurden. Der Träger kann beschichtet sein, beispielsweise mit Proteinen, insbesondere Antikörpern, Zuckern wie zum Beispiel Dextranen, oder aber mit Metallen, Glas, oder Kunststoffen, oder Carbonderivaten wie beispielsweise Carbon Nanotubes.

Im Sinne dieser Erfindung bedeutet "der Träger für eine Probenflüssigkeit teilweise durchlässig ist", dass die Probenflüssigkeit zumindest den Träger benetzt und eine teilweise Aufnahme der Probenflüssigkeit zumindest im Träger erfolgt. Ein geringes Durchtreten der Probenflüssigkeit ist ebenfalls nicht ausgeschlossen. Daher sind Membranen erfindungsgemäß bevorzugt und käuflich erhältlich.

Der Träger kann insbesondere zwei planparallele oder annähernd planparallele Oberflächen aufweisen, die erfindungsgemäß eine erste oder zweite Oberfläche ausbilden. Insbesondere kann der Träger die Form einer Membran oder eines flachen Balkens aufweisen. Die Träger können Nachweisplätze ausbilden und mit Rezeptormolekülen (Testreagenzien) versehen sein.

Ein oder mehrere Rezeptormoleküle sind auf der ersten Oberfläche des Trägers fixiert. Weiterhin kann mindestens ein Rezeptormolekül auf einer zweiten Oberfläche eines Trägers fixiert sein. Erfindungsgemäß können die Rezeptormoleküle auch in einer Schicht unterhalb der ersten oder zweiten Oberfläche fixiert sein. Die Rezeptormoleküle können zum Beispiel an oder nahe der Oberfläche eingetrocknet sein, entweder allein oder in einer stabilisierenden Matrix, oder als Lyophylisat aufgetragen werden und lösen sich bei Benetzung von der Oberfläche. Die Fixierung kann weiterhin durch Ausnutzung nicht kovalenter Wechselwirkungen erfolgen, wie sie beispielsweise zwischen Antikörper und Protein A bestehen, zwischen Biotin und Streptavidin, zwischen Hexa-Histidin und Nickel-NTA oder bei der Basenpaarung von DNA. Ferner kann ein Element der Rezeptormoleküle kovalent an die erste Oberfläche gebunden sein. In einer bevorzugten Ausführungsform bleibt das Rezeptormolekül bei Benetzung im Wesentlichen an der ersten Oberfläche fixiert, insbesondere zu mehr als 50%.

Eine Probenflüssigkeit im Sinne dieser Erfindung kann eine beliebige Substanz oder Substanzgemisch, ggfs. samt Lösungsmittel, beliebiger Herkunft sein, vorzugsweise jedoch eine biologische Flüssigkeit. Die Probenflüssigkeit kann insbesondere von einer Pflanze oder einem Tier stammen, insbesondere von einem Säugetier, insbesondere von einem Menschen. Die Probenflüssigkeit kann beispielsweise nicht abschließend sein: Vollblut, Halbblut, Serum, Speichel, Tränenflüssigkeit, Urin, Sekret, Hirnflüssigkeit oder prozessierte Formen solcher Flüssigkeiten, wie etwa EDTA - stabilisiertes Blut oder solche Flüssigkeiten sein, die diese vorgenannten Flüssigkeiten enthalten. Die Probenflüssigkeit kann ebenfalls verdünnt vorliegen. Die Probenflüssigkeit enthält mindestens einen Analyten der an mindestens ein Rezeptormolekül adressiert ist oder eine nachweisbare Wechselwirkung verursacht.

Die Probenflüssigkeit kann weiterhin Reagenzien enthalten, wie etwa Liganden, Kompetitoren, Antikörper oder markierte Antikörper, Enzyme oder Enzymsubstrate, insbesondere solche, die einen Farbumschlag oder eine Lumineszenz bewirken, und kann eine zweite organische Probeflüssigkeit enthalten, die beispielsweise einen anderen Antikörper enthält und einen Farbumschlag in einer weiteren Farbe bewirkt.

Eine weitere Lösung kann ferner Detergenzien enthalten, Fängermoleküle zum Binden freier Substanzen, die nicht an ein Rezeptormolekül gebunden haben, oder Enzyminhibitoren, so dass die Lösung zum Spülen oder Abstoppen einer Reaktion geeignet ist.

Eine Lösung kann bidestilliertes, destilliertes oder Leitungswasser mit oder ohne weitere Substanzen enthalten, es kann zusätzlich einen Ionenaustauscher durchlaufen haben. Ferner kann sie übliche Pufferkomponenten zur Stabilisierung des pH-Werts oder zur Stabilisierung von Bestandteilen der Probenflüssigkeit enthalten.

Die Probenkammer ist ein Hohlraum, in dem sich der Träger befindet, und beide Öffnungen (Einlass, Auslass) enthält. Die Probenkammer kann eine für die manuelle Handhabung optimierte Form aufweisen, beispielsweise in Form eines flachen Quaders, der in einer Hand gehalten werden kann. Bevorzugt ist der Quader weniger als 1 cm dick. Die Flüssigkeiten können nacheinander mit Spritzen, die mit der anderen Hand bedient werden, in die Probenkammer eingespritzt werden. Die Oberseite der Probenkammer kann einen transparenten Deckel enthalten, der den Blick zumindest auf Teile des Trägers freigibt und eine schnelle visuelle Auswertung des Tests gemäß dem erfindungsgemäßen Verfahren ermöglicht. Ferner kann die Probenkammer in der Weise gestaltet sein, dass sie in ein Lesegerät gelegt werden kann, dass Vorrichtungen zum Befüllen der Probenkammer oder die Auslesung der Tests enthält. Vorrichtungen dieser Art, insbesondere in miniaturisierter Form, können in einem weiteren Gehäuse oder dergleichen integriert sein.

Der Druck, mit dem die Probenflüssigkeit durch eine Öffnung in die Probenkammer fließt, kann vorzugsweise durch eine Spritze manuell erzeugt werden, wobei die Spritze bevorzugt ein Volumen aufweist, das nicht kleiner als ein Zehntel und nicht größer als das Zehnfache des Probenkammervolumens ist. Desweiteren ist bevorzugt, dass die Spritze einen Luer-Anschluss aufweist. Mit einer Spritze können die Flüssigkeiten zunächst aufgezogen und nachfolgend in die Probenkammer injiziert werden.

Die Spritze zur Druckerzeugung kann ebenfalls mit einer Spritzenpumpe betrieben werden. Vorteilhaft ist dabei, dass der Druck reproduzierbar und gleichförmig aufgebaut werden kann. Bei dieser Ausführungsform kann ein Schlauchverbinder zwischen Spritzenpumpe und der Probenkammer vorteilhaft sein, dessen kassettenseitiges Ende als Luer-Anschluss ausgestaltet sein kann. Weiterhin kann bevorzugt sein, ein Ventil mit mindestens zwei Wegen vorzusehen, so dass Reagenzien/Probenflüssigkeit aus einem oder mehreren Vorratsbehältern aufgezogen und nachfolgend in die Probenkammer abgegeben werden können.

Der Druck kann auch auf andere Weise manuell oder maschinell erzeugt werden, beispielsweise indem die Reagenzien/Probenflüssigkeit in einem kompressiblen und mit einer Öffnung versehenen Vorratsgefäß vorgehalten werden. Die Öffnung kann vorteilhaft mit einem Luer-Adapter versehen sein. Durch manuelle Druckausübung auf das Vorratsgefäß gelangt die Testlösung aus dem Vorratsbehälter in die Probenkammer. Der Druck auf den Vorratsbehälter kann auch maschinell erzeugt werden, beispielsweise indem ein Kolben, ein Hebel oder eine Quetschvorrichtung auf das Vorratsgefäß drückt, welches wiederum mit einem Motor, pneumatisch oder hydraulisch angetrieben werden kann.

Bei allen Verfahren und Vorrichtungen kann die Druckbeaufschlagung als Überdruck über der Einlassöffnung oder als Unterdruck über der Auslassöffnung anfallen.

Der Vorratsbehälter kann zur einmaligen Verwendung als Balg oder Blister ausgeführt sein, insbesondere in vorbefüllter und verschlossener Form. Vorteilhaft sind Bälge oder Blister aus Kunststoff oder Folie, insbesondere Einmalampullen. Es kann bevorzugt sein, Blister oder Einmalampullen in die Testkassette zu integrieren, so dass sie im gleichen Fertigungsgang gemeinsam hergestellt und die Vorratsbehälter in einem zweiten Schritt vorbefüllt werden.

In einer weiteren bevorzugten Ausführung der Erfindung wird eine Pumpe verwendet, die die Lösungen/Probenflüssigkeiten pumpt und in der Weise dem Probenraum zuführt. Die Pumpe kann sich zwischen Vorratsbehälter und Probenraum befinden und die Lösungen/Probenflüssigkeiten direkt pumpen, oder Luft oder eine Flüssigkeit in eine mit zwei Öffnungen versehenen Vorratsbehälter pumpen, so dass die darin befindliche Flüssigkeit verdrängt und in die Probenkammer gepumpt wird. Weiterhin kann vorgesehen sein, eine Pumpe zu verwenden, die vorwärts und rückwärts pumpen kann, und ein Ventil mit mindestens zwei Wegen zu verwenden, so dass Testreagenzien aus einem oder mehreren Vorratsbehältern herausgepumpt und nachfolgend in die Probenkammer abgegeben werden können. Die Pumpe kann sich auch hinter der Probenkammer befinden und Unterdruck in der Probenkammer erzeugen, so dass Flüssigkeiten aus einem kompressiblen Vorratsbehälter, der sich vor der Probenkammer befindet, durch Unterdruck in die Probenkammer gesogen und ggfs. weiter in den Abfallbehälter gesogen werden. Von besonderem Vorteil ist bei dieser Ausführungsform, dass die Pumpe mit keiner der Lösungen/Probenflüssigkeiten kontaminiert wird und daher ohne Reinigung weiter verwendet werden kann. Besonders geeignet sind Mikropumpen, wie beispielsweise O-run 100 oder O-run 200 von PARItec GmbH, Gräfelfing, Deutschland. Ferner sind Membranpumpen und Mikromembranpumpen, Schlauchpumpen und Zahnringpumpen besonders geeignet.

Eine Flüssigkeit, die eine Probenflüssigkeit oder eine Lösung sein kann, fließt in die Probenkammer mit einer Strömungskomponente parallel zur ersten und zweiten Oberfläche des Trägers. Befindet sich in der Flüssigkeit ein Analyt, der an mindestens einen Rezeptormolekül binden kann, so wird dies ermöglicht, sobald die Flüssigkeit mit dem Rezeptormolekül in Kontakt gebracht wird. Dabei kann es bevorzugt sein, nach dem Einbringen der Flüssigkeiten den Druck auf annähernd Atmosphärendruck zu entspannen, so dass die Flüssigkeiten auf dem Träger stehen, nicht oder kaum mehr strömt und Rezeptormolekül und Analyt inkubieren und binden können, ohne dass neue Flüssigkeit hinzugefügt werden muss. Ferner kann es bevorzugt sein, das Druckverhältnis eine Zeit lang umzukehren, so dass die Flüssigkeit sich in umgekehrter Richtung bewegt, oder die Druckverhältnisse eine Zeit lang zu alternieren, so dass die Flüssigkeit über dem Träger vor- und zurückbewegt wird. So ist im Vergleich zur unbewegten Flüssigkeit ein besserer Stoffaustausch zwischen Flüssigkeit und Träger möglich, ohne mehr Flüssigkeit zu benötigen.

Eine weitere Flüssigkeit/Lösung kann zum Spülen des Trägers verwendet werden. Auch beim Spülen kann es bevorzugt sein, die Flüssigkeit eine Zeit lang unbewegt oder schwach bewegt über dem Träger stehen zu lassen, um mit geringeren Flüssigkeitsmengen auszukommen. Ebenfalls beim Spülen kann der Druck alternierend beaufschlagt werden.

Überraschend ist, dass ein Testprotokoll wie das in Beispiel 1 beschriebene Resultate liefern kann, die mit dem bloßen Auge ähnlich gut auslesbar sind, wie beispielsweise aus dem Stand der Technik bekannten Lateral Flow Assays. Obwohl beim Lateral Flow Assay die Flüssigkeiten durch Kapillarkräfte durch den Träger und parallel zu seinen Oberflächen und folglich um die Rezeptormoleküle herum oder gar durch die Test-/Nachweisfelder hindurchfließen.

Ein Vorteil des erfindungsgemäßen Verfahrens ist weiterhin, dass es nicht unter Übersprechen leidet, das beim Lateral Flow Assay entstehen kann, wenn zwei auf unterschiedlichen Testfeldern befindliche Rezeptormoleküle an den gleichen Analyten binden.

Beim erfindungsgemäßen Verfahren geschieht das Überströmen des Trägers ausreichend schnell, sodass beim Kontakt mit der ersten Testreagenz keine wertverfälschende Depletion des Analyten aus der Flüssigkeit erfolgt und kein Einfluss auf die Wechselwirkung mit einem zweiten Rezeptormolekül auftritt.

Ein weiterer Vorteil des erfindungsgemäßen Verfahrens besteht darin, dass mehrere Flüssigkeiten nacheinander einfach, mit beliebigen zeitlichen Abständen und fast ohne Vermischung zugegeben werden können. Bei einem Lateral-Flow-Assay besteht die Möglichkeit dagegen ausschließlich mit Einschränkungen, da die Flüssigkeit lediglich mittels Kapillarwirkung des Teststreifens oder des Wickpads nachteilig bewegt wird. Einmal aufgetragen, bewegt sich eine Flüssigkeit kontinuierlich in Richtung des noch nicht benetzen Trägers oder Wickpads. Charakteristisch für die Kapillarwirkung ist weiterhin, dass gleichzeitig oder nacheinander auf einen Träger aufgetragene Flüssigkeiten ineinander laufen und sich vermischen. Infolge dessen können die sehr sensitiven enzymatischen Testverfahren, bei denen eine erwünschte Signalverstärkung beispielsweise über Meerrettichperoxidase oder alkalische Phosphatase eintritt, lediglich eingeschränkt durchgeführt werden, beispielsweise durch Eintrocknen. Tatsächlich beruhen kommerziell angebotene Lateral-Flow Tests ausschließlich auf Testprotokollen, bei denen kein enzymatischer Schritt vorkommt, und bei denen nicht mehr als ein Flüssigkeitsreagenz zur Probe zugegeben wird.

Auch gegenüber offen durchgeführten Tests, bei denen sich die Testlösungen im Überstand über einer Membran befinden, bestehen Vorteile. Bei diesen Tests wird eine freie oder in einem Rahmen fixierte Membran in einer Wanne manuell geschüttelt, wie es in EP1718970 der Anmelderin der Fall ist, oder die Wanne wird mit einem mechanischem Schüttler oder Taumelschüttler kontinuierlich bewegt. Durch das Schütteln wird eine Bewegung des Überstands relativ zur Membran induziert und folglich ein verbesserter Stoffaustausch für die Bindungsreaktion - wie auch für die Spülreaktion - erwirkt, der zu ähnlich guten Resultaten wie das erfindungsgemäße Verfahren führt. Die Vorteile des geschlossenen Aufbaus bestehen jedoch in der Vermeidung von Kontaminationen der Probe, dem Schutz des Anwenders vor Spritzern und Kontakt mit der Probeflüssigkeit, und der Unabhängigkeit von Betriebsparametern wie der Schüttelfrequenz des Schüttlers. Daher wird nach erfindungsgemäßen Verfahren die Reproduzierbarkeit verbessert

Überraschend ist ferner, dass die Resultate deutlich besser sind, wenn beide Oberflächen des Trägers mit den Flüssigkeiten in Kontakt kommen. Dies ist überraschend, da die zweite Oberfläche in beiden Fällen mit im Verhältnis ähnlichen Strömungen, Substanzmengen und Konzentrationen an Testflüssigkeit, Reagenzien und Spülflüssigkeiten in Kontakt kommt. Im experimentellen Vergleich wird jedoch eine deutlich schlechtere Spülwirkung beobachtet, wenn die zweite oder Rückseite des Trägers am Boden des Hohlraums befestigt ist.

Eine Auslesung des Testergebnisses kann dadurch erfolgen, dass die Testkassette einen transparenten Deckel aufweist, durch den die Testreagenzien beobachtet und Farbwert- oder Graustufenänderungen visuell ausgelesen werden können. Diese Anordnung ermöglicht auch eine fotometrische Auslesung des Testverfahrens, insbesondere bei Verwendung einer Optik, die das Testergebnis auf einen Bildsensor abbildet. Bevorzugt ist dieser Bildsensor ein CCD oder CMOS Sensor. Besonders kostengünstig und dennoch leistungsfähig sind Optiken aus dem Konsumerbereich, wie sie in Kameras, Webcams und Mobiltelefonen verwendet werden. Bei einem Test mit Farbumschlag oder Fluoreszenzänderung kann es bevorzugt sein, einen Farbfilter oder einen Farbsensor mit farbsensitiven Fotosensoren zu verwenden. Bei Verwendung von Fluoreszenz kann die Anregung der Fluoreszenz mit einer Lampe, einem Laser oder einer LED erfolgen.

In einer weiteren Ausführungsform werden elektrochemische Detektionsverfahren für die Auslesung verwendet, wie sie in den letzten Jahren allgemein für diagnostische Verfahren entwickelt wurden, so beispielsweise Dionex ED 40 Electrical Detector User Manual, http://www.dionex.com/en-us/webdocs/4529-34855-03.pdf, Gau, V. et al "Oral Fluid Nanosensor Test (OFNASET)with Advanced Electrochemical-Based Molecular Analysis Platform", Ann. N.Y. Acad. Sci. 1098: 401-410 (2007) doi: 10.1196/annals.1384.005. Wood, M. et al. "eSensor®: An Electrochemical Detection Based DNA Microarray Technology for Multiplexed Molecular Diagnostics" Abstracts of the 219th meeting of the Electrochemical Society (ECS) May 1, 2011 - May 6, 2011, Montreal, QC, Canada.

Die betrifft ebenfalls einen Kit bestehend aus einer Probenkammer enthaltend einen Träger samt fixierten Rezeptormolekülen zur Durchführung eines Bindungsassays mit einem Ein- und Auslass, wobei zur Kammerdecke und Kammerboden ein Freiraum besteht, ggfs. einer weiteren Nebenkammer, die mit der Probenkammer fluidisch verbunden ist, samt einem Mittel zur Druckbeaufschlagung und weitere Hilfs- und Zusatzstoffe, wie Stopplösung, Waschlösung etc. Weitere Ausgestaltungen können wie im vorstehenden Verfahren entsprechend vorgenommen werden.

Ein weiterer Gegenstand der Erfindung betrifft die Verwendung des erfindungsgemäßen Verfahrens oder eines Testsystems (Vorrichtung) zum Nachweis von mindestens einem Analyten (Substanzen) aus einer Probenflüssigkeit bei der human-, veterinärmedizinischen oder pflanzlichen Diagnostik, der Lebensmitteldiagnostik, der Umweltdiagnostik, der forensischen Diagnostik, der Pharmakologie, der Toxikologie, bei Allergie, Autoimmun- oder Stoffwechselerkrankungen, Infektionserkrankungen, Geschlechtserkrankungen, Nahrungsmittelunverträglichkeiten, parasitären Erkrankungen, Bestimmung von kleinen Molekülen wie Drogen, Medikamenten oder Stoffwechselprodukten, Zellmediatoren, Gewebetypisierung, Artentypisierung, Lebensmitteltypisierung, Antigentypisierung, Epitoptypisierung und DNA- oder RNA-Nachweis.

Besonders bevorzugt ist der Einsatz des erfindungsgemäßen Verfahrens oder eines Testsystems (Vorrichtung) im Point-of-Care Bereich.

### Beispiele und Figuren:

Diese Beispiele dienen ausschließlich zur Erläuterung der Erfindung, ohne die Erfindung auf diese Beispiele zu beschränken.

### Beispiel 1: Testprotokoll für Allergietest

### Vorbereitung

Testbox mit Röhrchen und Testkassette (Kit enthaltend Probenkammer) sowie die Patientenprobe auf Raumtemperatur (18-25°C) bringen.

Kurzzeitmesser, Einmalhandschuhe, Behälter für festen Abfall und Stift bereithalten.

Testkassette und Spritze entnehmen.

Alle Inkubationen erfolgen bei Raumtemperatur.

Die in der Testbox (Kit) eingesteckten Reagenzröhrchen entweder vorab oder sukzessive im Testverlauf aufrecht in die entsprechend farblich markierten Haltevorrichtungen am oberen Rand der Box umstecken.

### 1.1. Probeneinbringung:

Proberöhrchen (roter Deckel) mit zuvor eingebrachter Probe öffnen und den Inhalt mittels der Spritze vollständig und blasenfrei in die Testkassette überführen. Dazu Spritze in die Probenöffnung stecken und Inhalt zügig einspritzen.

Testkassette auf ebener Unterlage ablegen und 4 Minuten inkubieren.

### 1.2. Zugabe der Testlösung:

Wie zuvor (Abschnitt 10.1.) beschrieben Inhalt der Konjugatlösung (gelber Deckel) mit Hilfe der Spritze in den Einlass der Testkassette zügig einspritzen und diese abgelegt 8 Minuten inkubieren.

### 1.3. Waschen:

Erstes Röhrchen mit Waschlösung (blauer Deckel) öffnen und Inhalt wie zuvor beschrieben in Einlass der Testkassette zügig einspritzen. Unmittelbar anschließend den Inhalt des zweiten Röhrchens mit Waschlösung (blauer Deckel) zügig einspritzen. Keine Inkubation erforderlich!

### 1.4. Entwicklung:

Erstes Röhrchen mit Farbsubstrat (weißer Deckel) öffnen und Inhalt in Einlass der Testkassette zügig einspritzen. Unmittelbar anschließend das zweite Röhrchen mit Farbsubstrat (weißer Deckel) öffnen, Inhalt blasenfrei in Einlass der Testkassette einspritzen und diese abgelegt 8 Minuten inkubieren.

Während der Entwicklung werden 2 parallele Referenzstriche unterschiedlicher Intensität und gegebenenfalls zusätzlich ein zentraler Teststrich in jedem Testfeld sichtbar.

### 1.5. Stoppen:

Röhrchen mit Stopplösung (grüner Deckel) öffnen und Inhalt in Einlass der Testkassette einspritzen. Schieber der Testkassette schließen.

Hinweis:

Die Testresultate sind für mindestens 12 Stunden stabil und können in dieser Zeit abgelesen werden.

### Beispiel 2: Testkammer (Probenkammer)

Im Folgenden werden Maße für die Membran und ihre Abstände zu anderen Elementen der Vorrichtung angegeben. Dabei ist zu beachten, dass alle Maße für den trockenen Zustand der Membran angegeben werden, und dass eine mögliche Durchbiegung der Membran oder Oberflächenunebenheiten oder ein eventueller Verzug der Spritzgussteile nicht berücksichtigt werden. Im feuchten Zustand kann die Membran quellen oder durchbiegen und so eine veränderte Dicke und veränderte Abstände zu anderen Bauelementen aufweisen.

Eine (verstärkte) Nitrozellulosemembran von 140 µm Dicke wird auf ein Maß von 50,5 mm * 6,3 mm zugeschnitten. Sie wird zwischen zwei spritzgegossene Halbschalen geklemmt, die eine Probenkammer gemäß Abb. 1b und 2 bilden, deren Breite 3,5 mm beträgt. Das Gesamtvolumen der Membran beträgt damit 44,5 µl, das der freien Membran 24,7 µl. Der Abstand der Membranoberseite zur der im Wesentlichen planaren Innenseite der oberen Kammerwand beträgt 170 µm, der von der Membranunterseite zur im Wesentlichen planaren unteren Kammerwand der Halbschale 150 µm. Das Volumen der Kammer beträgt 81,3 µl. Der Querschnitt des nicht geklemmten Teils der Membran beträgt 0,49 mm², der der Kammer 1,62 mm².

Die Kammer ist mit einer Einlassöffnung in der Nähe eines Endes der Membran versehen, die einen Querschnitt von 0,3 mm² aufweist, und mit einer Auslassöffnung mit einem Querschnitt von 1,2 mm².

In der Testvorrichtung sind 4 Kammern parallel ausgeführt, so dass das gesamte Membranvolumen 98,98 µl beträgt, und das der Kammern ohne Zu- und Abläufe 325,22 µl. Sie werden gleichzeitig durch einen einzigen Zugang (Probenkanal) befüllt, welcher in 4 Kanäle auffächert, deren jeder mit einer Kammer verbunden ist. Die Auslassöffnungen laufen wiederum zu einem einzigen Kanal zusammen, der mit einem in die Testvorrichtung integrierten Waste-Volumen verbunden ist, in dem saugfähiges Material untergebracht ist. Das saugfähige Material ist fluidisch mit keiner Membran verbunden, so dass die Saugwirkung sich auf freie Flüssigkeit beschränkt, die in den Waste gelangt. Die Testvorrichtung weist Fenster auf, durch die die mit Reagenz beladenen Bereiche der Teststreifen beobachtet werden können.

Bei einem Test wird in die Testvorrichtung nach Protokoll 1 folgende Substanzen und Mengen dazugegeben:

| | |
|---|---|
| 1) Probe einschließlich Probenverdünner | 380 µl |
| 2) Waschpuffer je Vorgang | 1.000 µl |
| 3) Substratlösung je Vorgang | 1.000 µl |
| 4) Antikörper / Konjugatlösung | 600 µl |

Das Volumen jeder einzelnen Testkomponente ist größer als das Volumen der Membranen (100 µl).

### Beschreibung der Abbildungen:

Fig. 1a: Längsschnitt durch eine Probenkammer, bei der Flüssigkeit an beiden Oberflächen des Trägers (1) mit Rezeptormolekülen (2) im Fluss (3) parallel zu einander geführt werden. Die Kassette wird durch die Einlassöffnung (5) druckbeaufschlagt, so dass die Flüssigkeit von dort zum Waste (4) (Nebenkammer (supra)) fließt.
Fig. 1b: Längsschnitt durch eine Probenkammer, bei der Flüssigkeit am Ende des Trägers (1) rotiert wird und an der zweiten Oberfläche (Rückseite) antiparallel zur ersten Oberfläche fließt.
Fig. 2: Aufsicht auf eine Probenkammer. Es können mehrere Träger parallel in einer Kassette angeordnet werden. Dazu kann der Fluss aufgeteilt werden oder die Träger befinden sich gemeinsam in einem Hohlraum.

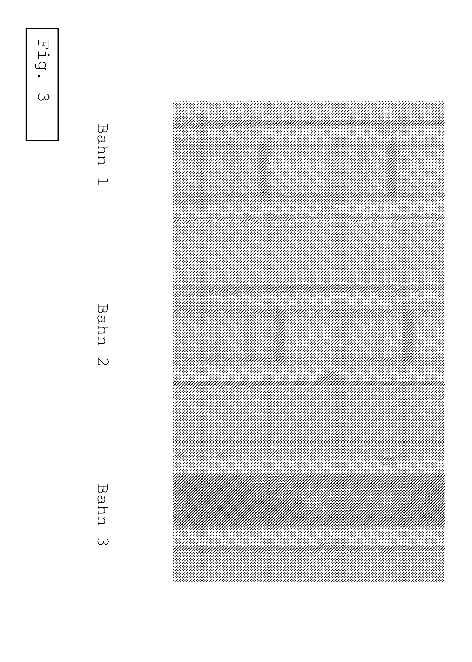

Fig. 3: Immunoasssay mit drei identischen Membranen als Festphase, zeitgleich durchgeführt nach Protokoll aus Beispiel 1. In Bahnen 1 und 2 sind die Membranen von beiden Seiten umströmt, in Bahn 3 wurde nur die Oberseite überströmt. In allen Bahnen wurden identische Reagenzien und Volumina verwendet.

## Patentansprüche

1. Testverfahren zum Nachweis von mindestens einem Analyten aus einer Probenflüssigkeit,
wobei in einer Probenkammer
a. mindestens ein Rezeptormolekül auf einer ersten Oberfläche eines Trägers fixiert ist, und rückseitig eine zweite Oberfläche ausgebildet ist,
b. der Träger für eine Probenflüssigkeit teilweise durchlässig ist,
c. über der ersten und zweiten Oberfläche jeweils zumindest ein Freivolumen ausgebildet ist, welches von einer Kammerwand begrenzt ist,
d. die Probenkammer mindestens zwei Öffnungen aufweist, wobei durch eine Öffnung eine Probenflüssigkeit entlang einem Strömungsgradienten über die erste und zweite Oberfläche zu der zweiten Öffnung fließt,
e. das Freivolumen aus c.) zumindest teilweise mit einer Flüssigkeitssäule ausgebildet ist,
f. wobei der Strömungsgradient entlang der ersten und zweiten Oberfläche mittels Druckbeaufschlagung erfolgt und die Flüssigkeitssäule aus d.) mitführt.

2. Testverfahren nach Anspruch 1, wobei der Abstand der ersten oder zweiten Oberfläche des Trägers zur Kammerwand 10 µm und mehr beträgt, insbesondere bis 1.500 µm.

3. Testverfahren nach Anspruch 2, wobei der Abstand 80 µm bis 350 µm, insbesondere 120 µm bis 200 µm, wobei ggfs. die Abstände der jeweils ersten Oberfläche oder zweiten Oberfläche des Trägers unterschiedlich sind.

4. Testverfahren nach einem der vorhergehenden Ansprüche, wobei der Träger aus einem festen Material besteht, ganz oder teilweise aus einer gelartigen, porösen, siebartigen, permeablen oder semipermeablen Membran, Dialysemembran, insbesondere einer beschichteten oder unbeschichteten Membran.

5. Testverfahren nach einem der vorhergehenden Ansprüche, wobei mindestens ein Rezeptormolekül auf einer zweiten Oberfläche eines Trägers fixiert ist.

6. Testverfahren nach einem der vorhergehenden Ansprüche, wobei der Strömungsgradient a.) parallel an der ersten und zweiten Oberfläche des Trägers geführt wird oder b.) antiparallel an der ersten und zweiten Oberfläche des Trägers geführt wird.

7. Testverfahren nach einem der vorhergehenden Ansprüche, wobei die Probenflüssigkeit eine biologische Flüssigkeit ist, insbesondere Vollblut, Halbblut, Serum, Speichel, Tränenflüssigkeit, Urin, Sekret, Hirnflüssigkeit oder prozessierte Formen solcher Flüssigkeiten, insbesondere EDTA - stabilisierte Flüssigkeiten.

8. Testverfahren nach einem der vorhergehenden Ansprüche, **dadurch gekennzeichnet, dass** die Druckbeaufschlagung mittels Über- oder Unterdruck erfolgt, insbesondere mit Hilfe einer Spritze, Ampulle, Pumpe oder Balg.

9. Testverfahren nach einem der vorhergehenden Ansprüche, **dadurch gekennzeichnet, dass** bei einer Eingabe einer Probenflüssigkeit von 80 - 120 µl die Probenkammer wie folgt gestaltet ist: Länge des Trägers 40 - 60 mm, Breite des Trägers 2,0 - 5 mm, Einlassdurchmesser 0,15 mm bis 0,45.

10. Testverfahren nach einem der vorhergehenden Ansprüche, **dadurch gekennzeichnet, dass** mehrere Probenkammern parallel aus einem Probenkanal mit mindestens einer Probenflüssigkeit versorgt werden.

11. Testverfahren nach einem der vorhergehenden Ansprüche, **dadurch gekennzeichnet, dass** Antikörper zum Nachweis des Analyten verwendet wird, der mit einem Enzym konjugiert oder assoziiert ist, insbesondere mit Meerrettichperoxidase oder alkalischer Phosphatase.

12. Kit bestehend aus einer Probenkammer enthaltend einen Träger samt fixierten Rezeptormolekülen zur Durchführung eines Bindungsassays mit einem Ein- und Auslass, wobei zur Kammerdecke und Kammerboden ein Freiraum besteht, ggfs. einer weiteren Nebenkammer, die mit der Probenkammer fluidisch verbunden ist, samt einem Mittel zur Druckbeaufschlagung und weitere Hilfs- und Zusatzstoffe.

13. Verwendung eines Kits nach Anspruch 12 oder eines Verfahrens nach einem der Ansprüche 1 bis 11 zum Nachweis von mindestens einem Analyten (Substanz) aus einer Probenflüssigkeit bei der human-, veterinärmedizinischen oder pflanzlichen Diagnostik, der Lebensmitteldiagnostik, der Umweltdiagnostik, der forensischen Diagnostik, der Pharmakologie, der Toxikologie, bei Allergie, Autoimmun- oder Stoffwechselerkrankungen, Infektionserkrankungen, Geschlechtserkrankungen, Nahrungsmittelunverträglichkeiten, parasitären Erkrankungen, Bestimmung von kleinen Molekülen wie Drogen, Medikamenten oder Stoffwechselprodukten, Zellmediatoren, Gewebetypisierung, Artentypisierung, Lebensmitteltypisierung, Antigentypisierung, Epitoptypisierung und DNA- oder RNA-Nachweis.
